# EUROPEAN PATENT SPECIFICATION

(11) **EP 2 919 763 B1**
(45) Date of publication and mention of the grant of the patent: **19.09.2018**
(21) Application number: 13855076.9
(22) Date of filing: 18.11.2013
(51) Int. Cl.: A61K 9/14, A61L 27/26, A61L 27/52, A61L 27/56, A61L 27/58

(54) **FLEXIBLE TISSUE MATRIX**
FLEXIBLE GEWEBEMATRIX
MATRICE DE TISSU FLEXIBLE

(30) Priority: 16.11.2012 US 201261727454 P
(43) Date of publication of application: 23.09.2015
(73) Proprietor: ISTO Technologies II, LLC, St. Louis, Missouri 63105 (US)
(72) Inventor: WARD, Anthony J., St. Louis, Missouri 63132 (US)
(74) Representative: Greaves Brewster LLP
(86) International application number: PCT/US2013/070573
(87) International publication number: WO 2014/078792

(56) References cited:
- WO-A2-2006/033698
- WO-A2-2007/143726
- US-A1- 2002 183 858
- US-A1- 2006 140 988
- US-A1- 2007 128 155
- US-A1- 2008 097 605
- US-A1- 2011 086 081
- US-A1- 2011 256 095
- OH ET AL: "In vitro and in vivo characteristics of PCL scaffolds with pore size gradient fabricated by a centrifugation method", BIOMATERIALS, ELSEVIER SCIENCE PUBLISHERS BV., BARKING, GB, vol. 28, no. 9, 14 January 2007 (2007-01-14), pages 1664-1671, XP005830614, ISSN: 0142-9612, DOI: 10.1016/J.BIOMATERIALS.2006.11.024
- ANDREW K EKAPUTRA ET AL: "The three-dimensional vascularization of growth factor-releasing hybrid scaffold of poly (-caprolactone)/collagen fibers and hyaluronic acid hydrogel", BIOMATERIALS, ELSEVIER SCIENCE PUBLISHERS BV., BARKING, GB, vol. 32, no. 32, 8 July 2011 (2011-07-08), pages 8108-8117, XP028277121, ISSN: 0142-9612, DOI: 10.1016/J.BIOMATERIALS.2011.07.022 [retrieved on 2011-07-14]
- CLAIRE G. JEONG ET AL: "Three-dimensional polycaprolactone scaffold-conjugated bone morphogenetic protein-2 promotes cartilage regeneration from primary chondrocytes in vitro and in vivo without accelerated endochondral ossification", JOURNAL OF BIOMEDICAL MATERIALS RESEARCH. PART A, vol. 100A, no. 8, 21 May 2012 (2012-05-21) , pages 2088-2096, XP055293400, HOBOKEN, NY, US ISSN: 1549-3296, DOI: 10.1002/jbm.a.33249

## Description

### TECHNICAL FIELD

The present disclosure relates generally to tissue matrices, scaffolds and supports for repair of biological tissues.

### BACKGROUND

Clinical demand exists for biocompatible matrices that offer tissue growth-conductive and or growth-inductive properties resembling those of autologous tissue. Additionally, need exists for such matrices that can be readily produced in large quantities, and are suitable for use in a variety of tissues including bone, cartilage, connective tissue and soft tissue.

Certain bone substitutes are available, but generally consist of materials that are characterized by poor resorption properties and poor response to physical manipulation, which complicate their use and radiographic evaluation. Other matrices have also been described, but demonstrate poor flexibility and are difficult to manipulate during a surgical procedure at the ambient temperatures typically encountered in surgical facilities. US 2008/097605 A1 and US 2002/183858 A1 disclose scaffolds comprising polycaprolactone and hyaluronic acid.

A need remains for biocompatible tissue matrices to aid in the repair of tissue such as bone, cartilage, connective tissue and/or soft tissue and which maintain structural integrity and are pliable and flexible when used during surgery.

### SUMMARY OF THE INVENTION

The scope of the invention is defined by the claims. Any references in the description to methods of treatment refer to the compounds, pharmaceutical compositions and medicaments of the present invention for use in a method for treatment of the human (or animal) body by therapy (or for diagnosis).

In one aspect, the present disclosure provides a matrix for supporting repair of biological tissues as defined in the claims; wherein the matrix is characterized by (i) retention of flexibility, compressive resistance and conformability when manipulated at temperatures typically found in operating theaters, (ii) the ability to support the growth of cells in vivo or ex vivo.

In another aspect, the present disclosure provides a flexible matrix for supporting repair of biological tissues as defined in the claims, optionally further comprising at least one flexibility agent entangled with the hyaluronic acid polymers, wherein the matrix is characterized by (i) retention of flexibility, compressive resistance and conformability when manipulated during a surgery, and (ii) the ability to support the growth of cells in vivo or ex vivo. In the matrix, the high molecular weight polymers are polycaprolactone, a co-polymer of polylactic acid and polycaprolactone, a co-polymer of polyglycolic acid and caprolactone, a copolymer of polycaprolactone and both polylactic acid and polyglycolic acid, or any combination thereof. In a non-limiting, exemplary tissue matrix, the high molecular weight polymers comprise polycaprolactone. The tissue matrix can further comprise polyester polymers entangled with the hyaluronic acid polymers. Polyester polymers can be polylactic acid, polyglycolic acid, a co-polymer of polylactic and polyglycolic acid, or any combination thereof. In a flexible matrix, total polymers can comprise high molecular weight polymers and any polyester polymers present, and the total polymers and the hyaluronic acid polymers can be combined in the matrix in a weight to weight ratio of from about 99:1 to about 1:99 (total polymers:hyaluronic acid polymers). In a non-limiting, exemplary tissue matrix, the total polymers and hyaluronic acid polymers are combined in the matrix in a weight to weight ratio of from about 5:1 to about 10:1.

In a flexible matrix as described herein, the hyaluronic acid polymers can be oxidized and/or covalently cross linked.

The high molecular weight polymers and polyester polymers can be prepared as a polymer blend. A flexible matrix can comprise, in non-limiting example, a polymer blend of polylactic acid and polycaprolactone, a polymer blend of polyglycolic acid and polycaprolactone, or a polymer blend of PLGA and polycaprolactone. Any of the additional polyester polymers can be combined with the high molecular weight polymers at a weight to weight ratio (polyester polymers : high molecular weight polymers) of from about 95:5 to about 1:9, preferably from about 9:1 to about 1:9, or more preferably from about 3:1 to about 7:3. In a tissue matrix comprising polylactic acid and polycaprolactone, the polylactic acid and the polycaprolactone can be combined in a polyester blend at a weight to weight ratio of from about 95:5 to about 1:9 (polylactic acid:polycaprolactone), more preferably from about 3:1 to about 7:3, and still more preferably about 7:3. In a tissue matrix comprising polyglycolic acid and polycaprolactone, the polyglycolic acid and the polycaprolactone can be combined in the polyester blend at a weight to weight ratio of from about 9:1 to about 1:9 (polycaprolactone + polyglycolic acid). In a tissue matrix comprising polylactic acid and a copolymer of (polycaprolactone + poly glycolic acid), the polylactic acid and the copolymer of polycaprolactone and poly glycolic acid) can be combined in a weight to weight ratio of from about 9:1 to about 1:9, preferably from about 3:1 to about 7:3. In a tissue matrix comprising PLGA and a copolymer of polylactic acid and polycaprolactone (PLCL), the PLGA and PLCL can be combined in a weight to weight ratio of from about 95:5 to 1:9, preferably at a weight to weight ratio of from about 9:1 to about 1:9.

Any matrix described herein can further include one or more flexibility agents and/or one or more growth factors, such as described herein. In a flexible matrix, total polymers can comprise high molecular weight polymers and any polyester polymers present, and the weight to weight ratio of total polymers to flexibility agents in the matrix can be from about 9:1 to about 99:1. A flexibility agent can be selected from triethyl citrate, acetyl tributyl citrate, acetyl triethyl citrate, tributyl citrate, trimethyl citrate, trihexyl citrate, acetyl trihexyl citrate, trioctyl citrate, acetyl trioctyl citrate or mixtures thereof. Alternatively, the matrix may further include a flexibility agent selected from polyethylene glycol, polyethylene glycol monoalkyl ether, propylene glycol, glycerin, triacetin or mixtures thereof. In a matrix including a flexibility agent selected from triethyl citrate, acetyl tributyl citrate, acetyl triethyl citrate, tributyl citrate, trimethyl citrate, trihexyl citrate, acetyl trihexyl citrate, trioctyl citrate, acetyl trioctyl citrate or mixtures thereof, the ratio of polymer to the flexibility agent can be for example about 9:1 to 99:1. In a matrix including a flexibility agent selected from polyethylene glycol , polyethylene glycol monoalkyl ether, propylene glycol, glycerin, triacetin or mixtures thereof, the ratio of polymer to the flexibility agent can be for example about 9:1 to 99:1. A matrix may include a combination of two flexibility agents, including for example one selected from triethyl citrate, acetyl tributyl citrate, acetyl triethyl citrate, tributyl citrate, trimethyl citrate, trihexyl citrate, acetyl trihexyl citrate, trioctyl citrate, acetyl trioctyl citrate or mixtures thereof, and one selected from polyethylene glycol , polyethylene glycol monoalkyl ether, propylene glycol, glycerin, triacetin or mixtures thereof, in which matrix the weight ratio of the polymer to the flexibility agents in combination can be from about 9:1 to about 99:1

### BRIEF DESCRIPTION OF THE DRAWINGS

FIG. 1 illustrates a introduction of a circular defect in a medial femoral condyle of a sheep, and introduction of a matrix into the defect.
FIG. 2 illustrates gross morphology in a medial femoral condyle of a sheep three months after introduction of a circular defect and a matrix implant.
FIG. 3 illustrates histological analysis of osteochondral tissue in a sheep three months after introduction of a circular defect and a matrix implant, including demonstration of hyaline cartilage and bone repair.
FIG. 4 illustrates endochondral bone formation at implant interface in a sheep three months after introduction of a circular defect and a matrix implant.
FIG. 5 illustrates gross morphology in a medial femoral condyle of a sheep six months after introduction of a circular defect and a matrix implant.
FIG. 6 illustrates histological analysis of osteochondral tissue in a sheep six months after introduction of a circular defect and a matrix implant, including demonstration of more extensive hyaline cartilage and bone repair compared to a femoral condyle three months after receiving a circular defect and a matrix implant.

### DETAILED DESCRIPTION

The present disclosure describes a new, improved tissue matrix for supporting tissue repair, which is as defined in the claims and optionally includes small molecule flexibility agents. Also described are methods for preparing the matrices, methods of using the matrices for promoting growth and repair of tissue, and use of the matrices for the manufacture of medicaments for supporting tissue repair.

### A. Definitions

Section headings as used in this section and the entire disclosure herein are not intended to be limiting.

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

The term "entanglement" and related terms, as used herein, refer to a state of polymers in melts or concentrated solutions above the overlap concentration, in which polymers interpenetrate one another and motion of the molecules is restricted to movement along a 'virtual tube' which surrounds each molecule, *i.e.* topological restriction of molecular motion by other molecular chains. For example, the term "reptation" has also been used to describe the thermal motion of long linear macromolecules that are entangled with one another in polymer melts or concentrated polymer solutions.

Methods described herein utilize laboratory techniques well known to skilled artisans, and guidance can be found in laboratory manuals such as Sambrook, J., et al., Molecular Cloning: A Laboratory Manual, 3rd ed. Cold Spring Harbor Laboratory Press, Cold Spring Harbor, N.Y., 2001; Spector, D. L. et al., Cells: A Laboratory Manual, Cold Spring Harbor Laboratory Press, Cold Spring Harbor, N.Y., 1998; and Harlow, E., Using Antibodies: A Laboratory Manual, Cold Spring Harbor Laboratory Press, Cold Spring Harbor, N.Y., 1999, and textbooks such as Hedrickson et al., Organic Chemistry 3rd edition, McGraw Hill, New York, 1970.

### B. Flexible Tissue Matrices

Tissue repair matrices consisting of certain entangled polyester-polysaccharides are described in U.S. Pat. Nos. 8,192,759 and 8,512,730. In contrast, the tissue matrices described herein improve substantially on tissue matrices consisting only of polylactic acid (PLA), polyglycolic acid (PGA), or a copolymer of PLA and PGA (poly(lactide-co-glycolide, PLA-PGA, or PLGA), entangled with hyaluronic acid.

Briefly, the inventors have successfully incorporated high molecular weight polycaprolactone polymers in tissue matrices. Surprisingly, the high molecular weight polycaprolactone polymers, when entangled with hyaluronic acid polymers as described herein, form matrices with the following beneficial aspects relative to a tissue matrix which is composed, for example, only of polyester polymers such as PLA, PGA or PLGA entangled with hyaluronic acid polymers: 1) increased flexibility at temperatures usually encountered in the operating theater (from about 18°C to about 21, 22, 23 or 24°C), while retaining the compressive resistant nature of the matrix; 2) increased aqueous absorption at physiological temperatures while maintaining a good dissolution profile relevant to the time scales for tissue regeneration; and 3) reductions in the appearance of a shiny surface on the strips of matrix, which in other matrices which incorporate only polyester polymers such as PLA, PGA, or PLGA result in migration of hyaluronic acid away from one surface during the manufacturing process. One or more flexibility agent(s) can be incorporated in the matrix to further enhance flexibility.

The observed improved flexibility of the tissue matrices described herein avoids or minimizes the need to further treat or manipulate the tissue matrix in the surgical environment. In contrast, a tissue matrix using only PGA, PLA or PLGA and lacking added flexibility agent(s) is relatively brittle under routine surgical conditions, and thus requires some form of additional heating to facilitate its use. Without being bound by theory, it is further believed that the increased absorbency of the improved tissue matrices described herein may be related to an improved ability for these matrices to imbibe and attach cellular component and further tissue formation.

A flexible tissue matrix for supporting repair of biological tissues comprises one or more high molecular weight polymers and optionally one or more flexibility agents, entangled with hyaluronic acid polymers. The flexibility agent(s) and high molecular weight polymers produce a matrix characterized by (i) retention of flexibility, compressive resistance and conformability when manipulated at temperatures typically found in operating theaters, (ii) the ability to support the growth of cells in vivo or ex vivo.

High molecular weight polymers are selected from, but are not limited to: polycaprolactone, co-polymers of polylactic acid and polycaprolactone (poly(lactide-co-caprolactone); co-polymers of polyglycolic acid and caprolactone (poly(glycolide-co-caprolactone); copolymers of polycaprolactone and both polylactic acid and polyglycolic acid; and any combination of the foregoing. Co-polymers can be obtained from a commercial supplier, or can be prepared according to well-known techniques, as described in references such as, in non-limiting example, Fukuzaki, Biomaterials 11: 441-446, 1990 and Jalil, J. Microencapsulation 7: 297-325, 1990. Thus, the high molecular weight polymer optionally is a high molecular weight co-polymer of polycaprolactone and any one or more of PLA, PGA or PLGA. A non-limiting example is a polymer blend of polycaprolactone and polyglycolic acid.

Flexible matrices can further include one or more polyester polymers such as any one of PLA, PGA, or a copolymer of polylactic acid and polyglycolic acid (PLGA), or any combination thereof. Any of the high molecular weight polymers described herein can be combined with one or more of the polyester polymers as a polymer blend.

Hyaluronic acid polymers are entangled with the other polymers by a process of dual solvent emulsion as detailed further below. The hyaluronic acid polymers can be oxidized or covalently cross linked. Cross-linkage can include any type of cross-linkage known to skilled artisans, for example as disclosed in references such as Laurent, T. C., Acta Chem. Scand. 18: 274-275, 1964; Kuo, J.-W Bioconjugate Chem. 2: 232-241, 1991; Mason, M., Biomaterials 21: 31-36, 2000; or Zhao, X. B., J. Mater. Sci. Mater. Med. 13: 11-16, 2002, and can include an aldehyde cross-linking agent such as formaldehyde or glutaraldehyde, a homobifunctional cross-linking agent or a heterobifunctional cross-linking agent such as a polysaccharide-reactive cross-linking agent. In various aspects, a cross-linkage can comprise oxidization, such as periodate-oxidization.

A flexible tissue matrix can be prepared at least from any one of the following combinations:
a) high molecular weight polymer(s) + hyaluronic acid polymers;
b) high molecular weight polymer(s) + flexibility agent(s) + hyaluronic acid polymers;
d) high molecular weight polymer(s) + polyester polymer(s) + hyaluronic acid polymers;
e) high molecular weight polymer(s) + flexibility agent(s) + polyester polymer(s) + hyaluronic acid polymers.

Total polymers can comprise one or more of the polyester polymers and one or more high molecular weight polymers combined in varying weight to weight ratios, from about 95:5 to about 1:9 (polyester polymers to high molecular weight polymers, by weight). Preferably, the weight to weight ratio of polyester polymers to high molecular weight polymers is from about 9:1 to about 1:9, and more preferably from about 3:1 to about 7:3. For example, a flexible tissue matrix may be prepared from total polymers consisting of polylactic acid and polycaprolactone combined in a weight to weight ratio of from about 95:5 to about 1:9 (polylactic acid to polycaprolactone, by weight), preferably from about 9:1 to about 1:9, and more preferably from about 3:1 to about 7:3.

A non-limiting example of a flexible matrix comprises polylactic acid blended with a copolymer of polylactic acid and polycaprolactone. Another non-limiting example of a flexible matrix comprises poly(lactide-co-caprolactone) (PLCL) blended with poly(lactide-co-glycolide) (PLGA).

Suitable flexibility agent(s) can be selected from, but are not limited to: triethyl citrate, acetyl tributyl citrate, acetyl triethyl citrate, tributyl citrate, trimethyl citrate, trihexyl citrate, acetyl trihexyl citrate, trioctyl citrate, acetyl trioctyl citrate, and any combination or mixture of any of the foregoing. Other suitable flexibility agents include, but are not limited to: polyethylene glycol, polyethylene glycol monoalkyl ether, propylene glycol, glycerin, triacetin, and any combination or mixture of any of the foregoing. When any one or more of the foregoing flexibility agents are incorporated in a matrix, the weight to weight ratio of total polymers to flexibility agent(s) described can be from about 9:1 to about 99:1. It should be understood that multiple flexibility agents can be incorporated in the matrix, in which case the weight to weight ratio of total polymers to total flexibility agents remains from about 9:1 to about 99:1.

A flexible matrix can incorporate at least one growth factor, such as, in non-limiting example: an epithelial growth factor (EGF), a vascular endothelial growth factor (VEGF), a member of the TGF-β superfamily, such as TGF-β1, TGF-β2, TGF-β3, or a bone morphogenetic protein (BMP); a growth differentiation factor; anti-dorsalizing morphogenetic protein-1 (ADMP-1); a fibroblast growth factor (FGF) such as acidic FGF or basic FGF; a member of the hedgehog family of proteins, such as indian hedgehog, sonic hedgehog, or desert hedgehog; an interleukin (IL); a colony-stimulating factor (CSF); an activin; a member of the insulin-like growth factor (IGF) family, such as IGF-I or IGF-II; a platelet-derived growth factor (PDGF) such as PDGF-AP, PDGF-BB and PDGF-AA; a member of the interleukin (IL) family, such as IL-1, IL-2, IL-3, IL-4, IL-5 or IL-6; or a member of the colony-stimulating factor (CSF) family, such as CSF-1, G-CSF, and GM-CSF. A growth factor can be obtained from a tissue source, or can be a recombinant growth factor produced in vitro, in a cell culture, or in a microorganism using standard molecular biology techniques. A growth factor can be a bone morphogenetic protein, such as, in non-limiting example, BMP-1, BMP-2, BMP-3, BMP-4, BMP-5, or BMP-6. In addition, a matrix can also include at least one collagen, such as, in non-limiting example, type I collagen, type IX collagen, type X collagen, or type XI collagen. The amount and species of a growth factor to add to a mixture can be determined by a skilled artisan by routine experimentation, and can be varied according to the intended use of a matrix.

A flexible matrix can also include one or more therapeutic agents, such as a pain medication, an anti-infective agent, an analgesic, an anti-inflammatory agent, or an immunosuppressive agent. Non-limiting examples of a therapeutic agent which can be comprised by a matrix include morphine, a nonsteroidal anti-inflammatory (NSAID) drug, oxycodone, morphine, fentanyl, hydrocodone, naproxyphene, codeine, acetaminophen, benzocaine, lidocaine, procaine, bupivacaine, ropivacaine, mepivacaine, chloroprocaine, tetracaine, cocaine, etidocaine, prilocalne, procaine, clonidine, xylazine, medetomidine, dexmedetomidine, and a VR1 antagonist. Suitable amounts of a therapeutic agent can be readily determined from resources known to those of skill in the art, and/or by routine experimentation.

Matrices can be prepared according to any of the weight to weight ratios of the components as follows. Total polymers, including high molecular weight polymers and any polyester polymers present, can be combined with hyaluronic acid polymers at a weight to weight ratio of from about 99:1 to about 1:99 (total polymers to hyaluronic acid polymers, by weight). A non-limiting, exemplary matrix in accordance with the present disclosure includes, for example, total polymers combined with hyaluronic acid polymers in a weight to weight ratio of from about 5:1 to about 10:1 (total polymers to hyaluronic acid polymers, by weight).

Flexible matrices are prepared using a dual solvent emulsion process. A selected amount (by weight) of a high molecular weight polymer is dissolved in an organic solvent such as ethyl acetate, dichloromethane or dichloroform. Routine blending techniques and equipment can be used. For example, blending can be achieved using a commercial blender for a period of one or more minutes. The high molecular weight polymer can be for example polycaprolactone, poly(lactide-co-caprolactone), poly(glycolide-co-caprolactone), or a copolymer of polycaprolactone and both polylactic acid and polyglycolic acid. Once the high molecular weight polymer is dissolved, a suitable amount (by weight) of any additional polyester polymer such as PLA, PGA or PLGA can be added to the solution and dissolved, thereby forming a polymer blend. Hyaluronic acid polymers (as hyaluronate, "HyA") are dissolved in water or optionally, prepared as a mixture with dry and wet ice which, when combined with the organic solution can cool the organic solution to about -40 ºC. Optionally, any one or more flexibility agent(s), growth factors and/or therapeutic agents can be incorporated in the matrix by adding an amount of any thereof to either the aqueous solution or the organic solution, depending upon the solubility characteristics of the selected flexibility agent(s), growth factor and/or therapeutic agents, as can be readily determined by one of average skill. The two solutions (the first organic solution of "total polymers" and the second, aqueous solution of HyA) are then combined, and mixed or agitated using any stirring or other mixing method as known in the art to form an emulsified mixture. The emulsified mixture is frozen. Following freezing, the emulsified mixture is lyophilized to remove the water and organic solvent, thereby yielding a solid, flexible matrix comprising the total polymers (high molecular weight polymer and any added polyester polymer), entangled with the hyaluronic acid. Alternatively, after blending, the emulsified mixture may be poured into a mold of any desired shape and brought to, e.g. about -10 ºC. The organic solvent then can be dissolved out of the mixture. Once all the organic solvent is driven out, the mixture may be freeze-dried to remove all water. Alternatively, instead of freezing and lyophilizing, the emulsified mixture can be wet laid and air dried. Membranes comprising a flexible matrix as described herein can further be prepared according to methods described in U.S. Patent No. 8,192,759, the disclosure of which is herein incorporated by reference. The dual solvent emulsion process entangles the molecules to produce a tissue matrix that a) achieves a wet strength sufficient to maintain the matrix's shape following hydration; b) it is interconnected, has a pore structure, and memory (i.e., it retains, regains pore structure and shape when hydrated, compressed and re-hydrated); and c) it supports, attracts and stimulates the growth of cells *in vivo* or *ex vivo* (*in vitro*).

The flexible tissue matrices can be used to promote tissue growth in a subject, such as but not limited to the implantation methods described in the examples provided herein below. A flexible matrix may be used alone to conduct the growth of tissue, in combination with at least one growth factor to induce the growth of tissue, in combination with cells to induce the growth of tissue, in combination with cells to induce the growth of tissue, and/or in combination with a collagen or fibrin, and/or in combination with one or more therapeutic agents.

A flexible tissue matrix can be introduced, *i.e.,* positioned at, implanted at or applied to a tissue site in need of tissue growth, in a subject in need thereof. A subject can be a mammal, such as, without limitation, a human, in particular a human patient in need of treatment, a companion animal, or a farm animal. In various configurations, a tissue can be, without limitation, bone, cartilage, tendon tissue, ligament tissue, soft tissue such as vascular tissue, dermal tissue or muscle tissue, or a combination thereof. In other configurations, a site in need of tissue growth can comprise tendon tissue, ligament tissue, vascular tissue, dermal tissue, periodontal tissue, intervertebral disc tissue, hyaline cartilage, fibrous cartilage, elastic cartilage, a nerve tunnel or a combination thereof. Accordingly, a mammalian subject can be a human patient in need of treatment, such as, in non-limiting example, an individual having an injury or degenerative disease of bone, cartilage, tendon tissue, ligament tissue, vascular tissue, dermal tissue, muscle tissue or a combination thereof. A skilled artisan such as a surgeon or a dermatologist can implant or inject a matrix at a site within the body of the patient.

The introduced flexible matrix can accelerate or promote the healing of tissue in contact with or adjacent to the flexible matrix. In non-limiting example, a human in need of treatment can be a patient having muscle atrophy or dystrophy resulting from a disease or disorder such as AIDS, diabetes, muscular dystrophy or cancer. In another non-limiting example, a subject in need of treatment can be a human in need of skin augmentation, such as, for example, a subject such as a patient having a dermal disorder such as skin folds due to aging and/or ultraviolet light exposure. An ameliorating treatment can comprise administering a matrix of the present teachings to the dermis of the patient, such as the patient's facial dermis. The administering can include, in non-limiting example, injection of an injectable form of a matrix adjacent to the skin targeted for skin augmentation. In yet another non-limiting example, a human in need of treatment can be a patient recovering from a traumatic injury to an internal organ which requires revascularization. In such an individual, growth of new blood vasculature can be directed and/or promoted by surgical implantation or injection of a matrix.

In various embodiments, the present teachings also encompass the use of a flexible matrix for the manufacture of a medicament for promoting tissue growth. Manufacture of a medicament can comprise the disclosed methods of forming a flexible matrix.

### C. Adaptations of the Methods of the Present Disclosure

By way of example, not of limitation, examples of the present invention shall now be given.

### EXAMPLES

### Example 1 (not according to the invention):

This example illustrates a method of constructing an entangled matrix comprising a polyester and a polysaccharide.

In this example, poly(lactide-co-glycolide) (PLGA) having molecular weight of about 1.5x105 is dissolved in dichloromethane (125 mg/ml), and Hyaluronate (HA) of molecular weight of about 1.3x106 Dalton is dissolved in water (15 mg/ml). The two polymer solutions, 2 parts PLGA, and 1 part HA, are mixed with 1 part Milli Q water by vortexing at high speed for about 5 minutes. The emulsified mixture is immediately poured into a mold pre-cooled at -70 ºC in a bath containing dry ice in isopropyl alcohol. After freezing, the mold and its contents are transferred into a second container that is loaded with dry ice and connected to a vacuum line. Organic solvent is removed by this process at the temperature between -70 ºC to -40 ºC, leaving HA in wet-ice phase. Water is then removed by raising the temperature to -10 ºC under vacuum, yielding a matrix comprising a polyester entangled with a hyaluronate polysaccharide.

### Example 2 (not according to the invention):

This example illustrates implant site preparation and post-implantation of a matrix in an animal model experimental system.

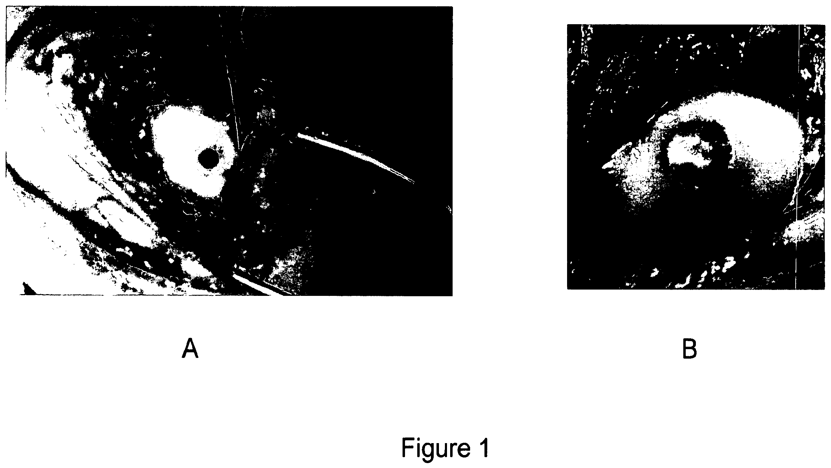

In this example, as shown in FIG. 1, a circular defect was created in the weight-bearing region of the medial femoral condyle of a skeletally mature sheep using a 5.5 mm diameter OATS-type punch to a depth of 6 mm in an animal model of an osteochondral defect (FIG. 1a). The defect was then flushed with sterile saline prior to insertion of a test matrix. An entangled polyester-polysaccharide matrix comprising PLA-PGA copolymer and hyaluronic acid as described in Example 1 was then press-fit into the defect using a blunt instrument. FIG. 1b shows the implant site post-implantation.

### Example 3 (not according to the invention):

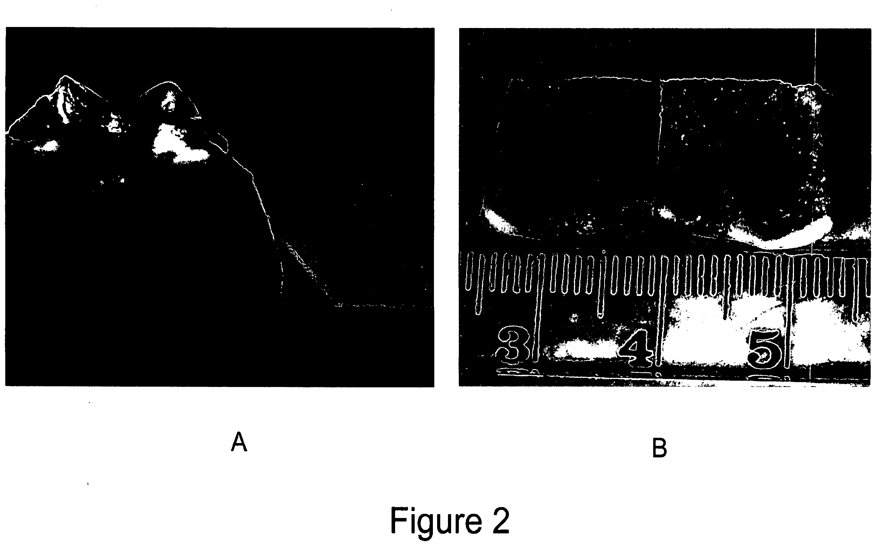

This example illustrates healing three months after the intervention illustrated in Example 2. FIG. 2 shows an example of gross outcome at 3 months. Gross image of the femoral condyle (FIG. 2a) and cross section of the tissue at the defect site (FIG. 2b) reveals new tissue formation fully integrated with the native cartilage and no evidence of inflammation at the site. FIG. 2b is a cross section of the implant site after decalcification showing extensive ingrowths and replacement of the implant with cartilage and bone. Animals having similar defects but not receiving an entangled polyester-polysaccharide matrix do not show the same extent of ingrowth of cartilage and bone.

### Example 4 (not according to the invention):

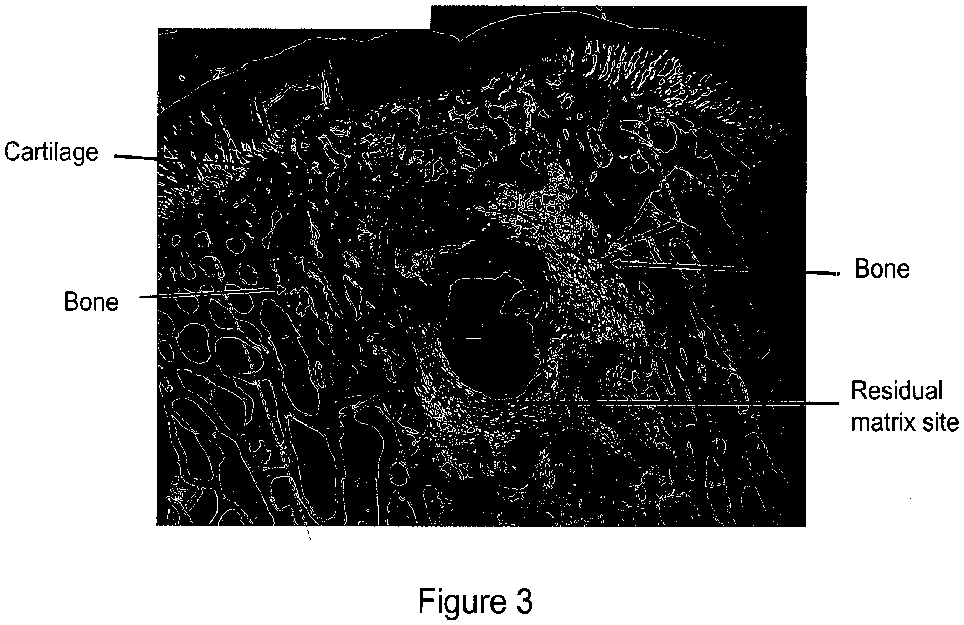

This example illustrates histological analysis of the 3-month post-intervention tissue presented in Example 3, and demonstrates hyaline cartilage repair with active osteogenesis at the core of the implant. In this example, as shown in FIG. 3, the tissue was stained with Safranin-O to visualize the cartilage proteoglycans found in the native and newly formed cartilage. FIG. 3 indicates that new cartilage and bone at the site of intervention become recognizable histologically upon examination 3 months following the procedure. FIG. 3 further indicates that greater than 50% of the composite implant was degraded/resorbed by 3 months post-intervention and was replace by newly formed bone and cartilage. Dashed line shows the marking of the surgical defect.

### Example 5 (not according to the invention):

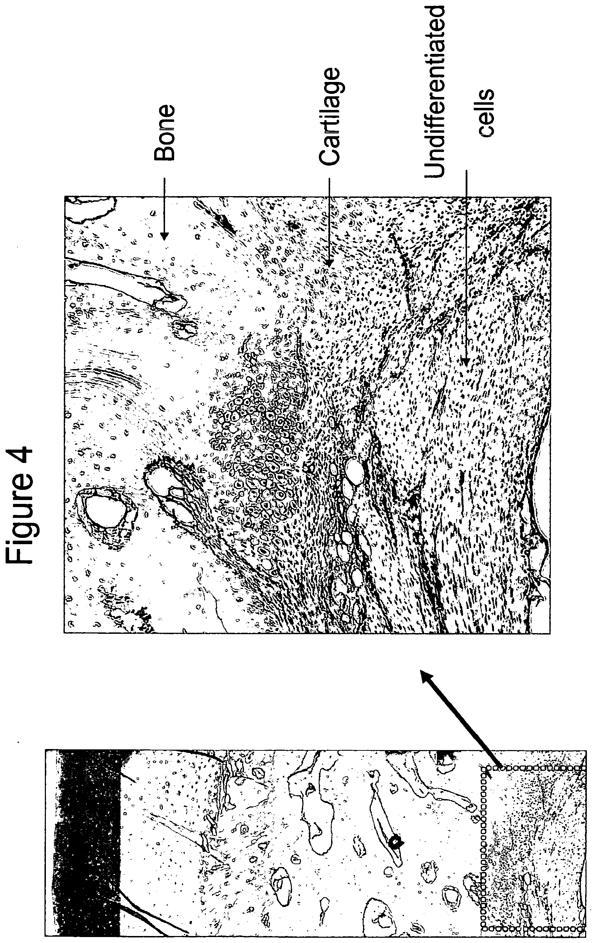

This example illustrates formation of cartilage and bone three months post-intervention. As shown in FIG. 4, light microscopy of an area close to the implant site reveals active formation of cartilage from undifferentiated stem cells derived from marrow cavity, and remodeling and formation of endochondral bone. Endochondral bone formation can be seen at implant interface.

### Example 6 (not according to the invention):

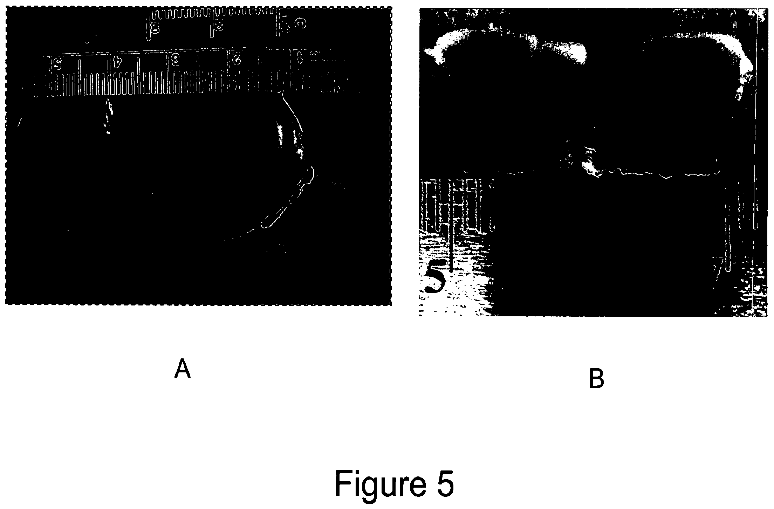

This example illustrates healing six months after the intervention illustrated in Example 2. FIG. 5 shows an example of gross outcome at 6 months post intervention. FIG. 5a shows gross images of the joint surface, while FIG. 5b shows a cross section of the tissue at the defect site. This figure demonstrates that healing of the osteochondral defect continued to improve from 3 to 6 months post-intervention. The healed tissue shows excellent integration and bonding with the native cartilage and bone, and over 90% of the implanted material is replaced with newly formed tissue.

### Example 7 (not according to the invention):

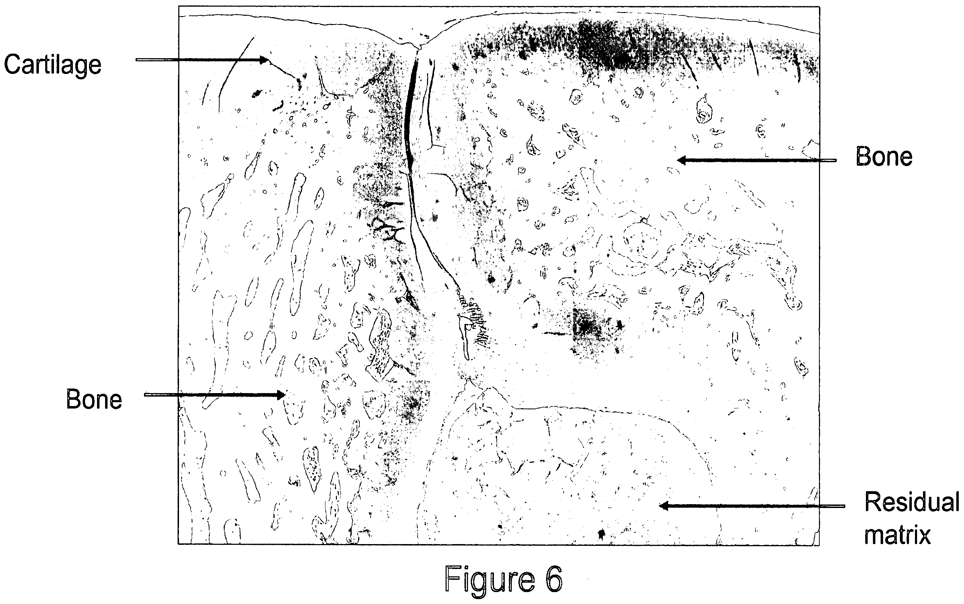

This example illustrates histological analysis of the 6-month post-intervention tissue presented in Example 6. As shown in FIG. 6, tissue was stained with Safranin-O to visualize staining of the cartilage proteoglycans found in the native and newly formed cartilage. FIG. 6 illustrates that greater than 90% of the implanted material was replaced with newly formed cartilage and bone.

### Example 8 (not according to the invention):

This example illustrates soft tissue contour augmentation. A patient presents with extensive facial wrinkles. An injectable formulation comprising an entangled polyester-polysaccharide matrix comprising PLA-PGA copolymer and hyaluronic acid is injected by a dermatologist within the dermis at sites of facial wrinkles. Improvements in appearance, i.e., diminishment of wrinkling, is observable to a casual observer within three months after injection.

### Example 9 (not according to the invention):

This example illustrates an anterior lumbar interbody fusion or (ALIF) spinal fusion using an interbody cage and a paste of the present teachings.

In this example, a trained individual such as a surgeon, inserts an interbody cage into the anterior aspect of the spine. The cage, which is a cylinder made of porous titanium, is inserted through a small incision (minilaparotomy), although an endoscope can also be used that allows the surgery to be done using a series of small incisions. Following insertion, the cage is filled with a paste comprising a matrix comprised of polyester entangled with hyaluronic acid, and further comprising a bone morphogenetic protein, recombinant human bone morphogentic protein-2 (rhBMP-2). The cage permits growth of a bone graft from a vertebral body through the cage, into the adjacent vertebral body. Patient recovery is significantly more rapid using this technique compared to a similar procedure omitting the matrix.

### Example 10 (not according to the invention):

This example illustrates formation of a paste. A mixture is formed, comprising 100 grams of granules of a matrix prepared as in Example 1 and an equal weight of an aqueous solution comprising hyaluronic acid (2% w/w). The components are blended in a microcutter for 10 minutes, yielding a viscous paste.

Example 11: In this example, poly(lactide-co-caprolactone) (PLCL) having a molecular weight of about 2 x 10⁵ is dissolved in ethyl acetate (80mg/ml). Once the PLCL is dissolved, poly(lactide-co-glycolide) (PLGA) having a molecular weight of about 1.5 x 10⁵ is added (240mg/ml) and dissolved. Hyaluronate (HyA) of molecular weight of about 1.5 x 10⁶ is dissolved in water (20mg/ml). The two polymer solutions, 3 parts PLGA/PLCL, and 2 parts HyA, are mixed and the emulsified mixture frozen. After freezing, the frozen emulsion is lyophilized to remove the two solvents yielding a matrix comprising PLGA/PLCL entangled with HyA.

One skilled in the art would readily appreciate that the articles and kits described in the present disclosure are well adapted to carry out the objects and obtain the ends and advantages mentioned, as well as those inherent therein.

## Claims

1. A flexible matrix for supporting repair of biological tissues comprising a high molecular weight caprolactone polymer entangled with hyaluronic acid polymers by a process of dual solvent emulsion, optionally further comprising at least one flexibility agent entangled with the hyaluronic acid polymers, wherein the matrix is **characterized by** (i) retention of flexibility, compressive resistance and conformability when manipulated during a surgery, and (ii) the ability to support the growth of cells in vivo or ex vivo, and wherein said dual solvent emulsion is formed by the steps of:
(i) dissolving the caprolactone polymer in an organic solvent;
(ii) dissolving the hyaluronic acid polymers in an aqueous solvent; and
(iii) blending the caprolactone polymer in the organic solvent with the hyaluronic acid polymers in an aqueous solvent to form the dual solvent emulsion.

2. A flexible matrix in accordance with claim 1, wherein the high molecular weight caprolactone polymers are selected from a group consisting of: polycaprolactone, a copolymer of polylactic acid and polycaprolactone, a co-polymer of polyglycolic acid and caprolactone, and a copolymer of polycaprolactone and both polylactic acid and polyglycolic acid.

3. A flexible matrix in accordance with claim 2, further comprising polyester polymers entangled with the hyaluronic acid polymers, wherein the polyester polymers are selected from the group consisting of polylactic acid, polyglycolic acid, and a copolymer of polylactic and polyglycolic acid, and wherein the high molecular weight caprolactone polymers and polyester polymers are prepared as a polymer blend.

4. A flexible matrix in accordance with claim 3, wherein total polymers comprise high molecular weight caprolactone polymers and any polyester polymers present, and wherein the total polymers and the hyaluronic acid polymers are combined in the matrix in a weight to weight ratio of from 99:1 to 1:99.

5. A flexible matrix in accordance with claim 4, wherein the total polymers and hyaluronic acid polymers are combined in the matrix in a weight to weight ratio of from 5:1 to 10:1.

6. A flexible matrix in accordance with claim 3, comprising a polymer blend of polylactic acid and polycaprolactone.

7. A flexible matrix in accordance with claim 6, wherein the polylactic acid and the polycaprolactone are combined in the polymer blend at a weight to weight ratio of from 95:5 to 1:9.

8. A flexible matrix in accordance with claim 6, wherein the polylactic acid and the polycaprolactone are combined in the polymer blend at a weight to weight ratio of 7:3.

9. A flexible matrix in accordance with claim 3, comprising a polymer blend of polyglycolic acid and polycaprolactone.

10. A flexible matrix in accordance with claim 9, wherein the polyglycolic acid and the polycaprolactone are combined in the polymer blend at a weight to weight ratio of from 9:1 to 1:9 (polycaprolactone + polyglycolic acid).

11. A flexible matrix in accordance with claim 2, wherein the polymer blend comprises polylactic acid and a copolymer of polyglycolic acid and polycaprolactone, and wherein the weight ratio of polylactic acid to the copolymer of polyglycolic acid and polycaprolactone is 3:1 to 7:3.

12. A flexible matrix in accordance with any one of claims 1-11, further comprising a flexibility agent, wherein the flexibility agent is selected from the group consisting of triethyl citrate, acetyl tributyl citrate, acetyl triethyl citrate, tributyl citrate, trimethyl citrate, trihexyl citrate, acetyl trihexyl citrate, trioctyl citrate, acetyl trioctyl citrate, polyethylene glycol, polyethylene glycol monoalkyl ether, propylene glycol, glycerin, triacetin and any combination thereof.

13. A flexible matrix in accordance with claim 11, wherein total polymers comprise high molecular weight polymers and any polyester polymers present, and the weight to weight ratio of total polymers to flexibility agents in the matrix is from 9:1 to 99:1.

14. A flexible matrix in accordance with claim 1, wherein the hyaluronic acid polymers are oxidized, and/or wherein the hyaluronic acid polymers are covalently cross linked.

15. A flexible matrix in accordance with claim 1, wherein the polymers comprise PLGA and a copolymer of polylactic acid and polycaprolactone (PLCL), wherein the weight to weight ratio of the combined PLGA and PLCL to the hyaluronic acid polymers is from 95:5 to 1:9.

## Patentansprüche

1. Flexible Matrix zum Unterstützen einer Reparatur von biologischen Geweben, umfassend ein Caprolactonpolymer mit hohem Molekulargewicht, verschränkt mit Hyaluronsäurepolymeren durch ein Zwei-Lösemittel-Emulsions-Verfahren, wahlweise ferner umfassend mindestens ein mit den Hyaluronsäurepolymeren verschränktes Flexibilisierungsmittel, wobei die Matrix **gekennzeichnet ist durch** (i) Beibehaltung von Flexibilität, Druckfestigkeit und Anpassbarkeit bei Bearbeitung während eines chirurgischen Eingriffs und (ii) die Fähigkeit, das Wachstum von Zellen *in vivo* oder *ex vivo* zu unterstützen, und wobei die Zwei-Lösemittel-Emulsion gebildet ist durch die Schritte:
(i) Lösen des Caprolactonpolymers in einem organischen Lösemittel,
(ii) Lösen der Hyaluronsäurepolymere in einem wässrigen Lösemittel und
(iii) Vermischen des Caprolactonpolymers in dem organischen Lösemittel mit den Hyaluronsäurepolymeren in einem wässrigen Lösemittel, um die Zwei-Lösemittel-Emulsion zu bilden.

2. Flexible Matrix nach Anspruch 1, wobei die Caprolactonpolymere mit hohem Molekulargewicht ausgewählt sind aus einer Gruppe, bestehend aus: Polycaprolacton, einem Copolymer aus Polymilchsäure und Polycaprolacton, einem Copolymer aus Polyglycolsäure und Caprolacton und einem Copolymer aus Polycaprolacton und sowohl Polymilchsäure als auch Polyglycolsäure.

3. Flexible Matrix nach Anspruch 2, ferner umfassend mit den Hyaluronsäurepolymeren verschränkte Polyesterpolymere, wobei die Polyesterpolymere ausgewählt sind aus der Gruppe, bestehend aus Polymilchsäure, Polyglycolsäure und einem Copolymer aus Polymilch- und Polyglycolsäure, und wobei die Caprolactonpolymere mit hohem Molekulargewicht und die Polyesterpolymere als Polymermischung hergestellt sind.

4. Flexible Matrix nach Anspruch 3, wobei Gesamtpolymere Caprolactonpolymere mit hohem Molekulargewicht und jegliche vorhandenen Polyesterpolymere umfassen, und wobei die Gesamtpolymere und die Hyaluronsäurepolymere in der Matrix in einem Gewicht-zu-Gewicht-Verhältnis von 99:1 bis 1:99 kombiniert sind.

5. Flexible Matrix nach Anspruch 4, wobei die Gesamtpolymere und die Hyaluronsäurepolymere in der Matrix in einem Gewicht-zu-Gewicht-Verhältnis von 5:1 bis 10:1 kombiniert sind.

6. Flexible Matrix nach Anspruch 3, umfassend eine Polymermischung aus Polymilchsäure und Polycaprolacton.

7. Flexible Matrix nach Anspruch 6, wobei die Polymilchsäure und das Polycaprolacton in der Polymermischung in einem Gewicht-zu-Gewicht-Verhältnis von 95:5 bis 1:9 kombiniert sind.

8. Flexible Matrix nach Anspruch 6, wobei die Polymilchsäure und das Polycaprolacton in der Polymermischung in einem Gewicht-zu-Gewicht-Verhältnis von 7:3 kombiniert sind.

9. Flexible Matrix nach Anspruch 3, umfassend eine Polymermischung aus Polyglycolsäure und Polycaprolacton.

10. Flexible Matrix nach Anspruch 9, wobei die Polyglycolsäure und das Polycaprolacton in der Polymermischung in einem Gewicht-zu-Gewicht-Verhältnis von 9:1 bis 1:9 (Polycaprolacton + Polyglycolsäure) kombiniert sind.

11. Flexible Matrix nach Anspruch 2, wobei die Polymermischung Polymilchsäure und ein Copolymer aus Polyglycolsäure und Polycaprolacton umfasst und wobei das Gewichtsverhältnis der Polymilchsäure zu dem Copolymer aus Polyglycolsäure und Polycaprolacton 3:1 bis 7:3 beträgt.

12. Flexible Matrix nach einem der Ansprüche 1 bis 11, ferner umfassend ein Flexibilisierungsmittel, wobei das Flexibilisierungsmittel ausgewählt ist aus der Gruppe, bestehend aus Triethylcitrat, Acetyltributylcitrat, Acetyltriethylcitrat, Tributylcitrat, Trimethylcitrat, Trihexylcitrat, Acetyltrihexylcitrat, Trioctylcitrat, Acetyltrioctylcitrat, Polyethylenglycol, Polyethylenglycolmonoalkylether, Propylenglycol, Glycerin, Triacetin und jede Kombination aus diesen.

13. Flexible Matrix nach Anspruch 11, wobei die Gesamtpolymere Polymere mit hohem Molekulargewicht und jegliche vorhandenen Polyesterpolymere umfassen und das Gewicht-zu-Gewicht-Verhältnis von Gesamtpolymeren zu Flexibilisierungsmitteln in der Matrix 9:1 bis 99:1 beträgt.

14. Flexible Matrix nach Anspruch 1, wobei die Hyaluronsäurepolymere oxidiert sind und/oder wobei die Hyaluronsäurepolymere kovalent vernetzt sind.

15. Flexible Matrix nach Anspruch 1, wobei die Polymere PLGA und ein Copolymer aus Polymilchsäure und Polycaprolacton (PLCL) umfassen, wobei das Gewicht-zu-Gewicht-Verhältnis des kombinierten PLGA und PLCL zu den Hyaluronsäurepolymeren 95:5 bis 1:9 beträgt.

## Revendications

1. Matrice flexible pour supporter la réparation de tissus biologiques, comprenant un polymère de caprolactone de poids moléculaire élevé enchevêtré avec des polymères d'acide hyaluronique par un procédé d'émulsion à double solvant, comprenant en outre optionnellement au moins un agent de flexibilité enchevêtré avec les polymères d'acide hyaluronique, la matrice étant **caractérisée par** (i) la rétention de sa flexibilité, de sa résistance à la compression et de sa conformabilité lorsqu'elle est manipulée au cours d'une intervention chirurgicale, et (ii) l'aptitude à supporter la croissance de cellules in vivo ou ex vivo, et dans laquelle ladite émulsion à double solvant est formée par les étapes consistant à :
(i) dissoudre le polymère de caprolactone dans un solvant organique ;
(ii) dissoudre les polymères d'acide hyaluronique dans un solvant aqueux ; et
(iii) mélanger le polymère de caprolactone dans le solvant organique avec les polymères d'acide hyaluronique dans un solvant aqueux pour former l'émulsion à double solvant.

2. Matrice flexible selon la revendication 1, dans laquelle les polymères de caprolactone de poids moléculaire élevé sont sélectionnés dans un groupe constitué de : polycaprolactone, un copolymère d'acide polylactique et de polycaprolactone, un copolymère d'acide polyglycolique et de caprolactone, et un copolymère de polycaprolactone et à la fois d'acide polylactique et d'acide polyglycolique.

3. Matrice flexible selon la revendication 2, comprenant en outre des polymères de polyester enchevêtrés avec les polymères d'acide hyaluronique, les polymères de polyester étant sélectionnés dans le groupe constitué de : acide polylactique, acide polyglycolique et un copolymère d'acide polylactique et polyglycolique, et les polymères de caprolactone de poids moléculaire élevé et les polymères de polyester étant préparés sous forme de mélange de polymères.

4. Matrice flexible selon la revendication 3, dans laquelle les polymères totaux comprennent des polymères de caprolactone de poids moléculaire élevé et tous polymères de polyester présents, et dans laquelle les polymères totaux et les polymères d'acide hyaluronique sont combinés dans la matrice dans un rapport poids/poids de 99:1 à 1:99.

5. Matrice flexible selon la revendication 4, dans laquelle les polymères totaux et les polymères d'acide hyaluronique sont combinés dans la matrice dans un rapport poids/poids de 5:1 à 10:1.

6. Matrice flexible selon la revendication 3, comprenant un mélange de polymères d'acide polylactique et de polycaprolactone.

7. Matrice flexible selon la revendication 6, dans laquelle l'acide polylactique et le polycaprolactone sont combinés dans le mélange de polymères dans un rapport poids/poids de 95:5 à 1:9.

8. Matrice flexible selon la revendication 6, dans laquelle l'acide polylactique et le polycaprolactone sont combinés dans le mélange de polymères dans un rapport poids/poids de 7:3.

9. Matrice flexible selon la revendication 3, comprenant un mélange de polymères d'acide polyglycolique et de polycaprolactone.

10. Matrice flexible selon la revendication 9, dans laquelle l'acide polyglycolique et le polycaprolactone sont combinés dans le mélange de polymères dans un rapport poids/poids de 9:1 à 1:9 (polycaprolactone + acide polyglycolique).

11. Matrice flexible selon la revendication 2, dans laquelle le mélange de polymères comprend l'acide polylactique et un copolymère d'acide polyglycolique et de polycaprolactone, et dans laquelle le rapport de poids d'acide polylactique au copolymère d'acide polyglycolique et de polycaprolactone est de 3:1 à 7:3.

12. Matrice flexible selon l'une quelconque des revendications 1-11, comprenant en outre un agent de flexibilité, l'agent de flexibilité étant sélectionné dans le groupe constitué de : citrate de triéthyle, acétylcitrate de tributyle, acétylcitrate de triéthyle, citrate de tributyle, citrate de triméthyle, citrate de trihexyle, acétylcitrate de trihexyle, citrate de trioctyle, acétylcitrate de trioctyle, polyéthylène glycol, éther monoalkylé de polyéthylène glycol, propylène glycol, glycérine, triacétine et toute combinaison de ceux-ci.

13. Matrice flexible selon la revendication 11, dans laquelle les polymères totaux comprennent des polymères de poids moléculaire élevé et tous polymères de polyester présents, et le rapport poids/poids des polymères totaux aux agents de flexibilité dans la matrice est de 9:1 à 99:1.

14. Matrice flexible selon la revendication 1, dans laquelle les polymères d'acide hyaluronique sont oxydés, et/ou dans laquelle les polymères d'acide hyaluronique sont réticulés par covalence.

15. Matrice flexible selon la revendication 1, dans laquelle les polymères comprennent le PLGA et un copolymère d'acide polylactique et de polycaprolactone (PLCL), dans laquelle le rapport poids/poids des PLGA et PLCL combinés aux polymères d'acide hyaluronique est de 95:5 à 1:9.
